# EUROPEAN PATENT SPECIFICATION

(11) **EP 0 564 893 B1**
(45) Date of publication and mention of the grant of the patent: **24.06.1998**
(21) Application number: 93104776.5
(22) Date of filing: 23.03.1993
(51) Int. Cl.: C12P 19/16, C07H 3/00

(54) **Amylase resistant starch**
Amylase-resistente Stärke
Amidon résistant à l'amylase

(30) Priority: 25.03.1992 US 857530; 29.12.1992 US 997794
(43) Date of publication of application: 13.10.1993
(73) Proprietor: National Starch and Chemical Investment Holding Corporation, Wilmington, Delaware 19809 (US)
(72) Inventor: Chiu, Chung-Wai, Westfield, New Jersey 07090 (US); Henley, Matthew, Somerset, New Jersey 08873 (US); Altieri, Paul, Belle Mead, New Jersey 08502 (US)
(74) Representative: Hagemann, Heinrich, Dr.rer.nat., Dipl.-Chem.

(56) References cited:
- WO-A-90/15147
- GB-A- 1 313 421
- US-A- 3 729 380
- US-A- 3 734 760
- JOURNAL OF CEREAL SCIENCE, vol. 4, no. 1, January 1986, London C.S.BERRY "Resistant Starch: Formation and Measurement of Starch that Survives Exhaustire Digestion with Amylolytic Enzymes during the Determination of Dietary Fibre"
- pages 301-314

## Description

This invention relates to a process for the manufacture of a starch product containing at least about 15% amylase resistant starch from a starch that contains at least 40% amylose, and the resistant starch obtained by that process.

It is known that the amylose fraction of starch retrogrades to form a starch resistant to alpha-amylase digestion, and that this resistant starch is beneficial as a dietary component contributing to the total dietary fiber content in food products. See, for example, C.S. Berry, "Resistant Starch: Formation and Measurement of Starch that Survives Exhaustive Digestion with Amylolytic Enzymes during the Determination of Dietary Fibre," Journal of Cereal Science 4 (1986) 301-314; and N-G. Asp, et al., "Formation of Enzyme Resistant Starch During Autoclaving of Wheat Starch: Studies in vitro and in vivo," Journal of Cereal Science 6 (1987) 159-172.

US-A-3,729,380 and US-A-3,734,760 disclose a method for preparing a low molecular amylose by gelatinizing starch, cooling it, and selectively hydrolyzing the branched parts in amylopectin with alpha-1,6-glucosidase. US-A-5,051,271, and WO 90/15147 call for a cycle, or repeated cycles, of heating and cooling to form an alpha-amylase resistant starch. These references state that since the retrogradation of amylose is retarded by the presence of amylopectin in the starch, the process can be accelerated by enzymatic conversion of amylopectin to amylose prior to retrogradation by the use of debranching enzymes, such as pullulanase or isoamylase, or by partial hydrolysis with alpha-amylase.

The starch material obtained by these methods is reported by Sievert and Pomeranz, Cereal Chemistry 67 (1990) 21, to exhibit a melting transition over two zones: one at 80°-107°C with a peak at 95°C, corresponding to the lipid complexation of amylose; the other at 120°-166°C with a peak at 153°C, corresponding to the amylase resistant fraction.

GB-A-1 313 421 discloses a process for producing an amylose mixture containing over 50% by weight of natural amylose by hydrolyzing a starch with α-1,6-glucosidase.

US-A-3 632 475 discloses a process for preparing long-chain amylose and short-chain amylose and separating one from another.

The invention provides a process for the production of a resistant starch product containing at least about 15% resistant starch that consists essentially of the steps of (a) preparing an aqueous slurry of a starch that contains at least 40% amylose at a solids content of 15% to 40%; (b) gelatinizing the starch slurry at a temperature of 120°-175°C; (c) adding an effective amount of a debranching enzyme to the gelatinized starch to hydrolyze the 1,6-glucosidic bonds of the starch molecules; (d) isolating the debranched resistant starch by drying or extrusion.

The invention also provides a process for producing the resistant starch product that, instead of step (d) above, comprises the steps of: (d) adding an effective amount of an inorganic salt to the debranched starch to precipitate the resistant starch product; (e) incubating the starch and salt mixture at temperatures between 50°-100°C; (f) collecting the precipitated resistant starch product.

The method does not require repeated cycles of gelatinization and incubation at low temperatures to produce the resistant starch product.

In another embodiment this invention is the thermostable, partially crystalline, debranched resistant starch product obtained by that process. The starch product is characterized by a melting endotherm with a peak in the range of about 115°-135°C, and amorphous regions characterized by a solubility in water at room temperature of greater than 7%. The starch product has a molecular weight in the range of 15,000-100,000 for the majority (greater than 80%) of the starch molecules.

The starches used in preparing amylase resistant starch may be derived from any source, for example, from corn, potato, sweet potato, wheat, rice, sago, tapioca, waxy maize, and sorghum. However, to obtain the high yields of this process, the preferred starting starch is a starch containing greater than 40% amylose, for example, HYLON V, a corn starch containing about 50% amylose, or HYLON VII, a corn starch containing about 70% amylose, both products of National Starch and Chemical Company, Bridgewater, New Jersey. The starch may be defatted or chemically modified, for example, converted, derivatized, or crosslinked, and still yield resistant starch.

The starting starch is dispersed into an aqueous slurry having a solids content of 15% to 40%, preferably about 15%, and heated at a temperature of 120°-175°C and sufficient pressure to effect gelatinization. Although gelatinization may be effected by any of the methods known in the art, the preferred method is to force the starch slurry through a jet cooker. Jet-cookers are well known in the industry and consist of a cooking chamber in which the starch slurry is contacted with live steam under elevated temperatures. The conditions for gelatinization are temperatures from 120°-175°C (250°-350°F), and pressures generally from 1.05 - 10.5 kg/cm² (15 -150 psi). Complete gelatinization is desired and is determined visually by the total disintegration of granular structure. The gelatinization process disrupts, in whole or in part, the associative bonding of the starch molecules within the raw starch granule. This prepares the starch molecules for debranching by making them more accessible to the debranching enzyme, resulting in more uniformly debranched starch molecules.

After the starch has been gelatinized, it is then prepared for enzymatic debranching. The starch solids content is adjusted to the highest feasible solids level (to keep the amount of water low and to facilitate subsequent drying of the starch), the preferred solids content being about 15%. A higher solids starch system may be employed if the starch is processed with adequate mixing to uniformly blend enzyme and starch at the higher solids.

After the solids content is fixed, the temperature and pH of the starch dispersion are readjusted to provide optimum enzyme activity. These parameters will vary depending upon the type and source of enzyme used, the enzyme concentration, the substrate concentration, and the presence or absence of inhibitors.

The preferred enzyme for the enzymatic debranching of this process is pullulanase (E.C. 3.2.1.41; pullulan 6-glucanohydrolase), a heat stable enzyme obtained from a species of Bacillus. Pullulanase will catalyze the hydrolysis of the alpha-1,6 linkages in pullulan and amylopectin, provided that there are at least two glucose units in the side chain. However, other endo-alpha-1,6-glucanohydrolases, such as isoamylase (E.C. 3.2. 1.68), or any other endo-enzyme that exhibits selectivity in cleaving the 1,6-linkages of the starch molecule, leaving the 1,4-linkages substantially intact, may be used to debranch starch according to this method.

When the enzyme used is the Bacillus pullulanase, and the starch solids content is in the range of 5% -35%, the reaction may be carried out in a pH range from 3.0 - 7.5, preferably from 4.5 - 5.5, and most preferred at 5.0. Buffers, such as acetates, phosphates, citrates, or the salts of other weak acids can be added to ensure that the pH will be at the optimum level throughout the debranching. At pH 5.0 the preferred temperature for the aqueous starch dispersion during the enzymatic debranching by the Bacillus pullulanase will be between 25° - 75°C, the more preferred being between 50° - 65°C, and the most preferred being 60°C. If shorter treatment times are desired, the optimum temperature range can be increased to 60° - 65°C (or higher, if the debranching enzyme is thermally stable at the higher temperatures), or a higher enzyme concentration can be used. As with other parameters of the enzyme reaction, the preferred and optimum temperature ranges will vary with changes in other parameters that affect enzyme activity, such as substrate concentration and pH, and these can be determined by the practitioner.

Optimum concentrations of enzyme and substrate are governed by the level of enzyme activity, which will vary depending upon the enzyme source, the enzyme supplier, and the concentration of the enzyme provided in commercially available batches. In general, the pullulanase enzyme is effective at 1500 PUN (pullulanlase units novo/kg starch) using a HYLON V or VII starch substrate at 15% solids content. Although the process of this invention makes use of an enzyme in solution, an enzyme immobilized on a solid support is intended to fall within the scope of this invention.

The enzymatic treatment is permitted to continue until essentially complete debranching has occurred, which in most systems under optimum enzymatic conditions will be in less than 24 hours. The enzyme is then deactivated by increasing the temperature of the starch dispersion tc at least 75°C for about 15 minutes, or alternatively, by adjusting the pH of the starch dispersion to below 3.0 and holding at that pH for about 1/2 hour.

After debranching and deactivation of the enzyme, the starch is recovered by extrusion processing, or by any method known and used in the art for drying starch, such as, spray drying, flash drying, air drying, freeze drying, vacuum drying, belt drying, drum drying. The extrusion process has been found to increase the yield of resistant starch and, therefore, it is the preferred method.

The extrusion apparatus can be any screw-type extruder, although the twin-screw extruder is preferred. A twin-screw extruder will typically have rotating screws in a horizontal cylindrical barrel with an entry port mounted over one end and a shaping die mounted at the discharge end. When twin screws are used, they may be co-rotating and intermeshing or non-intermeshing. Each screw will comprise a helical flight or threaded section and typically will have a relatively deep feed section followed by a tapered transition section and a comparatively shallow constant-depth meter section. The screws, which are motor driven, generally fit snuggly into the cylinder or barrel to allow mixing, heating and shearing of the material as it passes through the extruder.

Heat is provided through a channel, chamber or bore located in the barrel wall which contains an electrical calrod or coil, or a circulating heated medium, such as oil. The heat exchange means may also be placed in or along the shaft of the screw device. The starch is extruded at temperatures between 60°-180°C, preferably between 110°-180°C and temperature is controlled in zones along the length of the screw. Variations in any of the elements used in the extruder may be made as desired in accordance with conventional design practices in the field.

Alternatively, if the starch is recovered by precipitaion with salt, an inorganic salt is added to the starch dispersion and the mixture is incubated at 50° - 100°C, preferably 90° - 100°C. The salt can be any known salt that will not interfere with starch retrogradation and that will act to precipitate the resistant starch. Suitable salts are sodium sulfate, ammonium sulfate or magnesium sulfate, and sodium chloride, preferably are the sulfate salts, and are added to the deactivated starch slurry in a minimum of 10% of the solids content, preferably from 25% - 50%. The starch is collected, washed, and dried, and contains a minimum of about 15% resistant starch.

In addition, any of the embodiments of this process can be followed by acid or enzyme hydrolysis to remove from the starch product that portion of the starch that is not resistant starch and to give a starch product containing even higher levels of resistant starch.

If the end-use application requires purification of the starch product, the reaction impurities and by-products may be removed by dialysis, filtration, ion exchange processes, centrifugation or any other method known in the art for purifying the starch.

The resistant starch product prepared by this method shows a melting endotherm for the resistant fraction within the range of 115°-135°C, more typically within the range of 119°-123°C and a solubility of greater than 7% in water.

The following examples disclose procedures for the production of the starch products of this invention and an analytical comparison of those starches with starches produced by prior art methods. The samples were analyzed for dietary fiber by the Prosky method as outlined in the test procedures.

### Examples

**Reference Example 1**: Waxy corn starch (0% amylose) (1000g) was slurried into water and jet cooked at temperatures between 149°-160°C (300°-320°F). The starch was placed into a constant temperature bath set at 60°C and sodium benzoate (approximately 0.1% by weight of the starch) was added to the cooked starch as a preservative. The starch dispersion was diluted to a solids range of about 15%. The pH was adjusted to 5.0 with a solution of 3:1 water/concentrated HCI. Promozyme 200L (75ml), a commercial preparation of pullulanase, a product of NOVO-NORDISK, Danbury CT, was added when the starch temperature reached 60°C (140°F). The enzyme was allowed to debranch the starch for 48 hours and then was deactivated by lowering the pH to 2.7-3.0 with a solution of 3:1 water/concentrated HCI for 1/2 hour. The starch was neutralized to a pH of 5.0-5.5 with 3% NaOH in water. The product was collected as a dry powder in 100% yield by spray drying using an Anhydro portable spray dryer (Lab type, S1, Anhydro Corp., Copenhagen, Denmark) having an inlet temperature of 210°-215°C, and outlet temperature of 90°-100°C. A two fluid atomizing nozzle with air pressure at 2.10 kg/cm² (30 psi) was used to atomize the starch. The powder was found to contain 1.0% dietary fiber when assayed by the Prosky method.

### Inventive Examples

**Example 2**: Preparation of resistant starch using 100% amylose as the starting starch. Potato starch (100 parts, dry basis) and sodium sulfite (1 part) were added to 1300 parts of a 10% solution of magnesium sulfate (130 parts anhydrous magnesium sulfate in 1170 parts water). The pH was adjusted to 7.5 with 25% H₂SO₄. The slurry was then passed through a thermal converter with a pump pressure of 7.03 kg/cm² (100 psi), pump speed of 7.56 liters per min (2 gal/min), temperature of 158°C (315°F), with 1 minute dwell time, and the highest possible steam input (> 90%). After cooking, the percent solids were determined to be 14% (corresponding to 8.0% MgSO₄ and 6.0% potato starch). (At this point, the salt concentration should be within 8.0% - 9.5%, and if not in this range can be brought to this concentration by either dilution with water or addition of MgSO₄.) The solution was cooled to 20°C and allowed to age for 20 hours while being slowly agitated. After aging, the amylose was recovered by centrifuging and washed by repeated suspension in water followed by centrifuging. The product was air dried and dispersed at 15% solids in water. The slurry was jet cooked at about 155°C (310°C). It was debranched with 18.75 ml Promozyme following the method of reference example 1. The reaction was allowed to proceed for 24 hours, after which the enzyme was deactivated with acid. The sample was recovered by spray drying according to the method of example 1. This sample was found to contain 50.3% dietary fiber when analyzed by the Prosky method.

**Example 3:** Preparation of resistant starch using a high amylose starch, HYLON VII (a product of National Starch and Chemical Company, Bridgewater, New Jersey) as the starting material. HYLON VII starch (2kg) was slurried into water (11.31kg) and jet cooked at 149°-158°C (300°-315°F). The starch was placed into a constant temperature bath set at 60°C and sodium' benzoate (2g) was added as a preservative. The dispersed starch was adjusted to pH 5.0 with a solution of 3:1 water/concentrated HCl. Promozyme 200L (140ml) was added when the starch temperature reached 60°C (140°F). The enzyme was allowed to debranch the starch for 48 hours and then was deactivated by lowering the pH to 2.7-3.0 with a solution of 3:1 water/concentrated HCl for 1/2 hour. The starch was neutralized to a pH of 5.0-5.5 with 3% NaOH in water. The sample was divided into two parts. One part was spray dried according to the method of example 1. This sample was found to contain 25% dietary fiber when analyzed by the Prosky method.

**Example 4:** Preparation of resistant starch by extrusion techniques. The remaining part of example 3 was extruded on a Werner & Pfleiderer Type ZSK-30 twin-screw extruder. The screw configuration was 12-44 and was used with a 5 mm (x2) die. The screws were operated at a speed of 400-450 rpm and the barrel heating zones were set to 60°/100°/120°/150°/150°C. The barrel was placed under a vacuum of 35-40 cm Hg (14 - 16 inches Hg) through a single barrel vent. The extruded starch rope was air dried and ground to a fine powder. This sample was found to contain 30.0% dietary fiber when analyzed by the Prosky method.

**Example 5:** Preparation of resistant starch from chemically modified starches.
A. Acid converted starch. The product was prepared according to the method described in examples 1 except that National 78-0150 (1kg), a thin-boiling high amylose starch, available from National Starch and Chemical Company, Bridgewater, New Jersey, at 17% starch solids, was debranched with 75ml of Promozyme. After enzyme deactivation, the product was spray dried according to example 1. The RS level was found to be 20.8% by the Prosky method.
B. Esterified starch. HYLON VII starch was modified with 1.25% octenyl succinic anhydride under the following conditions: HYLON VII (800g) was slurried into 1200 ml of water. The pH of the slurry was raised to 7.4 with 3% NaOH. While maintaining the pH at 7.3-7.4, three increments of octenyl succinic acid anhydride, 3.3 ml each, were added with agitation 1/2 hour apart. When the reaction no longer consumed caustic, it was determined to be complete and the starch was collected by filtration.
   This esterified starch was then used as the base for preparing the starch product containing resistant starch. The product was prepared according to the method described in example 2 except that the reaction was carried out at 15% solids, the pH of the dispersion was 5.3, 37.50 ml of Promozyme was used, and no filtration step was employed. The reaction mix was spray dried, as is, following the enzyme deactivation step and the spray dried powder was found to possess 22.0% RS by the Prosky method.
C. Defatted starch. Granular HYLON VII was defatted by repeated extraction with methanol using a Soxeleht extractor for ten days. A portion of this defatted starch (500g) was debranched and spray dried. This sample was found to contain 32.4% dietary fiber when analyzed by the Prosky method.

**Example 6:** Recovery of resistant starch by salt precipitation. A debranched HYLON VII suspension was prepared according to the method of example 2 and MgSO₄ was added in an amount equivalent to 25% by weight of the solids of the suspension. The salt and starch suspension was heated to 95°C and held at that temperature for 24 hours. The sample was cooled to about 25°C and ethanol was added to bring the solvent to a solution of 50:50 ethanol water and to precipitate out the starch product. The product was filtered over a Buchner funnel, washed twice with 50:50 ethanol:water and air dried. The sample was found to contain 40.9% dietary fiber when analyzed by the Prosky method. Similar yields of rsistant starch were obtained using other salts. The results are set out in the following table.

| **Salt Induced Formation of Resistant Starch** | | |
|---|---|---|
| Salt | Concentration | % RS |
| ------ | 0 | 22.6 |
| (NH₄)₂SO₄ | 25% | 40.8 |
| Na₂SO₄ | 10% | 33.8 |
| Na₂SO₄ | 25% | 42.1 |
| MgSO₄ | 25% | 40.9 |
| NaCl | 25% | 33.3 |
| NaCl | 50% | 44.0 |

**Example 7:** Production of a purified resistant starch product. Debranched HYLON VII was prepared according to the method of example 2 except that the product was not filtered. Instead 7.5ml of the reaction mix was placed in a boiling water bath and heated to 90°C. Termamyl 120L, a heat stable alpha-amylase produced by NOVO-NORDISK, (7.5ml) was added to the starch and the reaction mix incubated at 85° - 90°C for 24 hours. The insoluble product was isolated by filtration in 60% yield and was dried under ambient conditions by crumbling the filter cake and spreading it thinly on a drying rack. The dried particles were ground to a fine powder. The powder was analyzed by the Prosky method and found to contain 65% RS.

### Comparative Examples

**Example 8:** The production of resistant starch from processes known in the art.
A. Method of WO 90/15147 of Pomeranz et al. HYLON VII starch (2 kg) was slurred into water (8.65 kg) and poured into #1 retort canning jars. The starch was retorted at 126°-127°C (260°F) for two hours, and cooled in the retort to 25°C. The starch was then cooled at 4°C for 72 hours. The starch was brought to room temperature, removed from the retort cans, broken to a smaller size and slurried in deionized water to a concentration of about 10% starch solids. This slurry was further comminuted using an Eppenbach homogenizer on high speed. The pH of the slurry was adjusted to 6.3 with 3% NaOH in water and the slurry heated to 90°-95°C in a boiling water bath. Termanyl 120L (alpha-amylase, a product of NOVO) was added (5ml per 100 grams starch) and the enzyme and starch slurry incubated for 60 minutes at that temperature. The enzyme was deactivated by lowering the pH to 3.0 with a solution of 3:1 water/concentrated HCl for 1/2 hour. The starch was neutralized to a pH of 5.0-5.5 with 3% NaOH in water. The mixture was centrifuged and the insoluble components recovered by decanting off the supernatant; this step was repeated after resuspending the insolubles in deionized water. The insolubles were further isolated by resuspension in deionized water followed by filtration over Whatman 54 paper in a Buchner funnel. The insoluble product was air dired and ground to a fine powder. This sample was found to contain 47% dietary fiber when analyzed by the Prosky method.
B. Method of US-A-5,051,271 High amylose starch (150 g) was suspended in 400 ml of water to make a smooth, homogenous slurry. This slurry was added to 2.6 L of boiling (autoclaved) water with constant stirring. The resulting suspension was autoclaved at 121°C for 8 hours. The solution was cooked and incubated at 24°C for 16 hours and then at 8°C for 120 hours. Resistant amylose that precipitated during this process was removed from the suspension by repeated centrifugation and resuspended at 10% solids with stirring to form a smooth, homogenous suspension. The 10% starch solids suspension was then subjected to enzymatic hydrolysis. Two enzyme suspensions were prepared separately: 1.4 g of HT (HT-proteolytic, Solvay Enzymes, Elkhart, IN) concentrated alpha-amylase were added to 26.6 ml of water, and 2,500 U human salivary amylase (Sigma Type 1X-A) were added to 100 ml deionized water. Both of the amylase solutions were then added to the starch suspension and the reaction mixture stirred at 23°C for 24 hours. The resulting enzyme-modified resistant starch (EMRS) was removed from the suspension by repeated centrifugation. The solid was dispersed in water to form a 10% (w/w) suspension and freeze-dried. This sample was found to contain 52% dietary fiber when analyzed by the Prosky method.
C. Method of US-A-3,734,760 A 15% amylomaize starch suspension (1 liter) (amylose content 70%) was gelatinized by heating with agitation at 165°C for 10 minutes in a pressure cooker (Buechiglasuster, Fabik-Nr 106703,1644 1644 HBZ). The cooked starch was cooled rapidly to 60°C. Thereafter, purified isoamylase (EC 3.2.1.68) enzyme derived from Pseudomonas (Sigma Chemical, cat.# I.2758), 125,000 units, was added to the gelatinized solution and incubated at 45°C for two days in a reciprocating incubator. The amylose mixture was cooled to -5°C and precipitated out. The starch was recovered by freeze-drying (Dura-Top MP, FTS Systems, Stone Ridge, NY). This sample was found to contain 12.2% dietary fiber when analyzed by the Prosky method.

**Example 9:** Preparation of resistant starch from 100% amylose without debranching. The amount of resistant starch obtained from 100% amylose without debranching is considerably less than obtained from 100% amylose with debranching (see example 2). A portion of example 2 after jet cooking, but without debranching, was allowed to cool to room temperature with good agitation, and then held at room temperature for 20 hours to allow the amylose to retrograde. The amylose was collected as a dry powder by spray-drying. It was analyzed for RS by the Prosky method and found to contain 24.6% RS.

**Example 10:** Effect of alpha-Amylase on resistant starch formation. This example illustrates that the addition of alpha-1,4 specific enzymes to a starch dispersion before retrogradation may be detrimental to the formation of RS.

HYLON VII starch (2kg) was slurried into water (11.31kg) and jet cooked at 149°-158°C (300°-315°F). The starch was placed into a constant temperature bath set at 80°C and sodium benzoate (2g) was added to the cooked starch to preserve freshness. The dispersed starch was adjusted to pH 6.0 with a solution of 3:1 water/concentrated HCl. The batch was split evenly into two portions and each batch maintained at 80°C. Bacterial alpha-amylase (BAN 120L, a product of Novo-Nordisk, Danbury CT) was added at levels of 0.005% and 0.01%, based on the weight of starch, to the two fractions, respectively. The first fraction was degraded to a viscosity of 100 mPas (cps) (at 10% solids, 21.5°C) and the second to a viscosity of 30 - 50 mPas (cps) (at 10% solids, 21.5°C). The alpha-amylase was then deactivated by adding 3:1 water/concentrated HCl until the dispersion was adjusted to pH 3.0 and held for 1/2 hour. The temperature of the starch was lowered to 60°C and the pH adjusted to 5.0. The debranching enzyme, pullulanase, Promozyme (65 ml), was added to each fraction and allowed to react for 48 hours. The pullulanase was deactivated and the insolubles collected by resuspending in water and spray drying. The two products were analyzed for resistant starch by the Prosky method and were found to contain 17.5% and 1.8% RS respectively.

**Example 11:** Effect of amylose level in starch base on resistant starch production. This example shows that the yield of resistant starch increases as the amount of amylose in the base starch increases.

All of the samples were gelatinized and debranched according to the procedure outlined in example 2 from the base starches and in the amounts and enzyme concentrations as follows:
A. Waxy corn starch (0% amylose) (1000g) was used as the base. Promozyme (75ml) was used to debranch the starch. The product was collected as a dry powder in 100% yield by spray drying; no filtration step was used. The powder was found to contain 0% resistant starch when assayed by the Prosky method.
B. Corn starch (25% amylose) (500g) was used as the base. Promozyme (37ml) was used to debranch the starch. The product was collected as a dry powder in 100% yield by spray drying; no filtration step was used. The powder was found to contain 0% resistant starch when assayed by the Prosky method.
C. HYLON V, a high amylose corn starch (about 50% amylose), (500g) was used as the base. Promozyme (35ml) was used to debranch the starch. The product was collected as a dry powder in 100% yield by spray drying; no filtration step was used. The product was found to contain 14.3% resistant starch when assayed by the Prosky method.
D. HYLON VII, a high amylose corn starch (about 70% amylose), (500g) was used as the base. Promozyme (37ml) was used to debranch the starch. The product was collected as a dry powder in 100% yield by spray drying; no filtration step was used. The powder was found to contain 28.8% resistant starch when assayed by the Prosky method.

The results are set out in the following table.

| **Effect of Amylose Content on Yields of Resistant Starch** | | |
|---|---|---|
| Sample | % Amylose | % RS Yield |
| 11-A | 0 | 0 |
| 11-B | 25 | 0 |
| 11-C | 50 | 14.3 |
| 11-D | 70 | 28.8 |
| 2 | 100 | 50.3 |

**Example 12:** Comparison of Drying Methods. This example shows that efficient high temperature drying may be used to recover the starch product containing resistant starch.

The starch product was prepared according to the method of example 2 except that the starch used was 10kg of HYLON VII; the debranching reaction was carried out using 750ml Promozyme; and the starch was dispersed at 11% solids. The insoluble product was isolated by centrifugation using a perforated bowl centrifuge with a linen cloth as the filter medium. The cake obtained from the centrifuge was divided into two fractions.
A. One fraction was re-slurried and spray dried using the method and conditions of example 1. The dry powder obtained was analyzed by the Prosky method and found to contain 25% resistant starch.
B. The other portion was flash dried using a laboratory flash dryer having an inlet temperature of 250°C (480°F) and an outlet temperature of 175°C (350°F) at a feed rate of 7.0 g/min. The dry powder obtained from this process was analyzed by the Prosky method and was found to contain 30.6% resistant starch.
C. A sample was prepared according to the procedure in example 2 except that the starch used was 3kg of HYLON VII starch and the conversion was carried out for 72 hours. The insoluble product obtained by filtration was crumbled, spread thinly and allowed to dry under ambient conditions. The dry particles were recovered and ground to a fine powder. The powder was analyzed by the Prosky method and was found to contain 26.0% resistant starch.
The results are set out in the following table.

| **Effect of Method of Drying on RS Production** | | | |
|---|---|---|---|
| Example | Method | Drying Temp. °C | % RS |
| 12-A | Spray | 260 | 25.0 |
| 12-B | Flash | 250 | 30.6 |
| 12-C | Air | 25 | 26.0 |

### Test and Analysis Procedures

DSC procedure. DSC measurements were performed on each of the samples with a Perkin-Elmer DSC-4 instrument equipped with a 3600 thermal analysis data station and a Perkin-Elmer graphics plotter 2 (Perkin-Elmer Corporation, Instrument Division, Norwalk, CT). Samples of ∼12 mg were weighed accurately into Perkin-Elmer stainless steel pans. About 40 µl of deionized water were added and the pans were sealed and allowed to equilibrate overnight to 4°C. The DSC scan was run from 50°-180°C at 10°C/min heating rate. An empty pan represented the reference sample. The peak transition temperature (Tₚ) was defined and read as the temperature at the peak maximum.

Solubility procedure. Solubility was determined by measuring the difference in the optical rotation of a 0.5% starch dispersion and of the supernatant of the dispersion after centrifugation on a Perkin-Elmer #141 Polarimeter equipped with a sodium lamp.

The starch was first blended in a Waring blender equipped with a semi-micro stainless steel cup. Approximately 30-40 ml of distilled water were added into the blender cup. With the blender on low speed, approximately 0.50 gram of starch (as is) was dispersed into the water within 15-30 seconds. The speed was Increased to high and the starch agitated for 2 minutes. The solution was transferred to a 50 ml volumetric flask and diluted to mark with distilled water. This was designated the stock solution. The stock solution was shaken well and divided into two portions by removing 25 ml by pipet. The stock solution remaining was reshaken, dispersed with 10 ml of 5N KOH, and diluted to the 50 ml mark with distilled water. The 25 ml sample removed by pipet was transferred to a 50 ml centrifuge tube, and spun down in an International Model K centrifuge at 1800-2000 rpm for 15 minutes. Then 12.5 ml of supernatant was transferred by pipet to a 25 ml volumetric flask, 5ml of 5N KOH was added with swirling, and the supernatant diluted to the 25 ml mark with distilled water. The optical rotation of both the stock solution and the supematant sample solution were measured in a 0.998 cm polarimeter cell and the percent water solubles determined by the following equation.$\text{% Water Solubles =} \frac{\text{Optical Rotation of Supernatant (α)/Path Length of Supernatant}}{\text{Optical Rotation of Stock Solution (α)/Path Length of Stock Solution}} \text{X 100}$

Digestibility Determination. Digestibility was determined by measuring the difference in the concentration of a 0.5% starch dispersion and of the supernatant of the dispersion after digestion with alpha-amylase and centrifugation. The optical rotation was measured on a Perkin-Elmer #241 Polarimeter equipped with a 1.002cm cell, a continuous sodium lamp, a continuous Na/589 filter/source, having a recorder range of +/- 5 degrees and an integration speed of 0.1 seconds. A 0.5ml aliquot of Slgma-A6255 Lot 60H8045 porcine pancreas alpha-amylase (16,800 units) was dissolved into 14.5 ml of 0.5M phosphate buffer at pH 7.5. A one gram sample of starch and 5 ml of enzyme solution were dispersed into a 50ml volumetric flask, diluted to mark with the buffer solution. The dispersion was transferred to a 50 ml Erlenmeyer flask, the flask was placed in a shaker/incubator at 37°C for two hours. A 2.5 ml sample was removed, diluted to 0.5% solids with buffer solution, and centrifuged at 2000 rpm for 10 minutes. A specimen of the supematant was removed and the optical rotation measured. The percent digestibility determined by the following equation in which 203 is the constant for the rotation of starch in water.$\text{% Digestibillity =} \frac{\text{Optical Rotation of Supernatant (α)/Path Length of Supernatant X 203}}{\text{0.005}} \text{X 100}$

Molecular Weight Determination. Molecular weight was determined by gel permeation chromatography. Samples were prepared for analysis by slurrying 5 mg starch in 4 ml of dimethylsulfoxide (DMSO) containing 0.03M sodium nitrate and heating the slurry to 80°C for at least 30 minutes. Samples In the amount of 200 microliters were injected into an ALC/GPC-150C Chromatograph (Waters Associates, Milfor, MA) equipped with a Nelson 3000 Series Chromotography Data System and two PL gel mixed 10 micromol columns (obtained from Polymer Laboratory, Amherst, MA) employing DMSO containing 0.03M sodium nitrate as the mobile phase, and eluted at a rate of 1 ml/min. The column was calibrated using dextran standards with molecular weights of 2,000, 20,000, 80,000, and 500,000 (obtained from Pharmacia Fine chemicals, Piscataway, NJ). The molecular weight is recorded as the peak molecular weight integrated by the data system.

Total Dietary Fiber Determination. This example outlines the Prosky Method for determining dietary fiber in foods according to Prosky, et al., J. Assoc. Off. Anal. Chem., **68**, 677 (1985).
Reagents:
   (a) Ethanol 95% v/v, technical grade.
   (b) Ethanol 78%. Place 207ml H₂O into 1 L volume flask. Dilute to volume with 95% EtOH. Mix and dilute to volume again with 95% EtOH if necessary. Mix.
   (c) Acetone, reagent grade.
   (d) Phosphate buffer, 0.05M, pH 6.0. Dissolve 0.875g Na phosphate dibasic, anhydride (Na₂HPO₄) (or 1.097g dihydrate) and 6.05g Na phosphate monobasic monohydrate (NaH₂PO₄) (or 6.8g dihydrate) in ca 700ml H₂O. Dilute to 1 L with H₂O. Check pH with pH meter.
   (e) Termamyl (heat stable α-amylase) solution - No. 120 L, Novo Laboratories, Inc., Wilton Connecticut 06897. Keep refrigerated.
   (f) Protease. No. P-5380, Sigma Chemical Company. Keep refrigerated.
   (g) Amyloglucosidase. No. A-9268, Sigma Chemical Company. Keep refrigerated. Alternatively, a kit containing all 3 enzymes (pretested) is available from Sigma Chemical Company, Catalog No. KR-185.
   (h) Sodium hydroxide solution, 0.171N. Dissolve 6.84g NaOH ACS in ca 700ml H₂O in 1 L volume flask. Dilute to volume with H₂O.
   (i) Phosphoric acid solution, 0.205M. Dissolve 23.64g H₃PO₄ ACS (85%) in H₂O in 1 L volume flask. Dilute to volume with H₂O.
   (j) Celite C-211, acid-washed. Fisher Scientific Company.
Method:

Run blank through entire procedure along with samples to measure any contribution from reagents to residue.

Homogenize sample and dry overnight in 70°C vacuum oven, cool in desiccator, and dry-mill portion of sample to 0.3 - 0.5mm mesh.

Weigh duplicate 1g samples, accurate to 0.1mg, into 400ml, tall-form beakers. Sample weights should not differ by >20mg. Add 50ml pH 6.0 phosphate buffer to each beaker. Check pH and adjust if necessary. Add 0.1ml Termanyl solution. Cover beaker with Aluminum foil and place in boiling H₂O bath 15 minutes. Shake gently at 5 minute intervals. Increase incubation time when number of beakers in boiling H₂O bath makes it difficult for beaker contents to reach internal temperature of 100°C. Use thermometer to ascertain that 100° is attained at 15 minutes. Total of 30 minutes in H₂O bath should be sufficient.

Cool solutions to room temperature. Adjust to pH 7.5 ± 0.1 by adding 10ml 0.171N NaOH solution.

Add 5 mg protease. (Protease sticks to spatula, so it may be preferable to prepare enzyme solution just before use with ca 0.1ml phosphate buffer and pipet required amount).

Cover beaker with aluminum foil. Incubate 30 minutes at 60°C with continuous agitation. Cool. Add 10ml 0.205M H₃PO₄ solution to adjust pH to 4.5 ± 0.2. Add 0.3ml amyloglucosidase, cover with aluminum foil and incubate 30 minutes at 60°C. (Measure volume before heating.) Let precipitate form at room temperature for 60 minutes.

Weigh crucible containing Celite to nearest 0.1mg, then wet and redistribute bed of Celite in crucible by using stream of 78% EtOH from wash bottle. Apply suction to draw Celite onto fritted glass as even mat Maintain suction and quantitatively transfer precipitate from enzyme digest to crucible.

Wash residue successively with three 20ml portions of 78% EtOH, two 10ml portions of 95% EtOH, and two 10ml portions of acetone. Gum may form with some samples, trapping liquid. If so, break surface film with spatula to improve filtration. Time for filtration and washing will vary from 0.1 - 6 hours, averaging 1.2 hour per sample. Long filtration times can be avoided by careful intermittent suction throughout filtration.

Dry crucible containing residue overnight in 70°C vacuum oven or 105°C air oven. Cool in desiccator and weigh to nearest 0.1mg. Subtract crucible and Celite weight to determine weight of residue.

Analyze residue from sample of set of duplicates for protein and ash. Subtract protein and ash values from residue to obtain TDF.
Determination of blank:$\text{blank = mg blank residue -} \frac{\text{(% protein in blank + % ash in blank) x mg blank residue}}{\text{100}}$
Determination of TDF (%):$\text{TDF% = mg residue -} \frac{\text{[(% protein in residue + % ash in residue) x mg residue] - blank}}{\text{mg sample (wt)}} \text{x 100}$

Total dietary fiber contents, DSC peak temperatures, solubility data, digestibility data and molecular weights for the samples are set out in the following table. Example 1 containing no amylose exhibits a peak melting transition temperature at 78°C; example 2 comprising 100% amylose exhibits a peak melting transition temperature at 132°C. Examples 3, 4, 5C, and 6 show a peak transition temperature in the range of 119°-123°C, corresponding to the debranched amylose of example 2. Examples 8A and 8B, containing 70% amylose, were not debranched but were prepared by methods known in the art and exhibited peaks at about 150°C. The data show that the resistant starch products made by debranching exhibit a melting endotherm distinct from the peaks for starch products previously reported.

| Characteristics of Resistant Starch Products | | | | | | | | |
|---|---|---|---|---|---|---|---|---|
| Example | % amylose | treatment | recovery | TDF % by wt | Digestibility | DSC peak °C | MWx 1000 | Solubility % In H₂O |
| 1 | 0% amylose | debranched | spray-dried | 1.0 | 52.2 | 78 | 3-300 | 42.5 |
| 2 | 100% amylose | debranched | spray-dried | 50.3 | 18.7 | 132 | 143-146 | 0.0 |
| 3 | 70% amylose | debranched | spray-dried | 25.0 | 27.2 | 121 | 15-100 | 15 |
| 4 | 70% amylose | debranched | extruded | 30.0 | 22.6 | 124 | 17-70 | 9.1 |
| 5C | 70% amylose defatted | debranched | spray-dried | 32.4 | --- | 119 | --- | --- |
| 6 | 70% amylose | debranched | salt ppt. 25% MgSO₄ | 40.9 | 22.7 | 122 | 17-50 | 7.0 |
| 8A | 70% amylose | hydrolyzed not debranched | ppt | N/A | 11.6 | 151 | 12-15 | 4.3 |
| 8B | 70% amylose | hydrolyzed not debranched | ppt/freeze-dried | N/A | 5.9 | 149 | 11-12 | ≤1.0 |
| 8C | 70% amylose | debranched | ppt/freeze-dried | 12.2 | 25.6 | 101 | 100-156 | 0.0 |

## Claims

1. A process for the production of a resistant starch product containing at least about 15% resistant starch that consists essentially of the steps of:
(a) preparing an aqueous slurry of a starch that contains at least 40% amylose at a solids content of 15% to 40%;
(b) gelatinizing the starch slurry at a temperature of 120°-175°C;
(c) adding an effective amount of a debranching enzyme to the gelatinized starch to hydrolyze the 1,6-glucosidic bonds of the starch molecules;
(d) isolating the debranched resistant starch by drying or extrusion.

2. The process according to claim 1 in which the starch in step (a) is a defatted starch or a chemically modified starch.

3. The process according to claim 1 in which the debranching enzyme is selected from the group consisting of pullulanase and isoamylase.

4. The process according to claim 1 in which the debranched starch in step (c) is further treated by acid or enzyme hydrolysis before isolating the resistant starch product.

5. A process for the production of a resistant starch product containing at least about 15% resistant starch that consists essentially of the steps of:
(a) preparing an aqueous slurry of a starch containing at least 40% amylose at a solids content of 15% to 40%;
(b) gelatinizing the starch slurry at a temperature of 120°-175°C;
(c) adding an effective amount of a debranching enzyme to the gelatinized starch to hydrolyze the 1,6-glucosidic bonds of the starch molecules;
(d) adding an effective amount of an inorganic salt to the debranched starch to precipitate the resistant starch product;
(e) incubating the starch and salt mixture at temperatures between 50° - 100°C;
(f) collecting the precipitated resistant starch product.

6. The process according to claim 5 in which the starch in step (a) is a defatted starch or a chemically modified starch.

7. The process according to claim 5 in which the debranching enzyme is selected from the group consisting of pullulanase and isoamylase.

8. The process according to claim 5 in which the inorganic salt is added to the debranched starch in an amount of 10% - 50% by weight of the starch solids.

9. The process according to claim 5 in which the inorganic salts are selected from the group consisting of ammonium sulfate, sodium sulfate, magnesium sulfate, and sodium chloride.

10. The process according to claim 5 in which the crystallized starch product of step (a) is further treated by acid or enzyme hydrolysis before isolating the resistant starch product.

11. A resistant starch product characterized by a melting endotherm having a peak in the range of 115°-135°C.

12. The resistant starch product according to claim 11 further characterized by a solubility in water at room temperature of greater than 7%.

## Patentansprüche

1. Verfahren zur Herstellung eines mindestens etwa 15 % resistente Stärke enthaltenden resistenten Stärkeprodukts, das im wesentlichen aus den Schritten
(a) Herstellen eines wäßrigen, einen Feststoffgehalt von 15 bis 40 % aufweisenden Breis einer Stärke, die mindestens 40 % Amylose enthält;
(b) Gelieren des Stärkebreis bei einer Temperatur von 120°-175 °C;
(c) Zugeben einer wirksamen Menge eines entzweigenden Enzyms zur gelierten Stärke, um die 1,6-glucosidischen Bindungen der Stärkemoleküle zu hydrolysieren;
(d) Isolieren der entzweigten resistenten Stärke durch Trocknung oder Extrusion
besteht.

2. Verfahren nach Anspruch 1, wobei die Stärke in Schritt (a) eine entfettete Stärke oder eine chemisch modifizierte Stärke ist.

3. Verfahren nach Anspruch 1, wobei das entzweigende Enzym aus der aus Pullulanase und Isoamylase bestehenden Gruppe ausgewählt wird.

4. Verfahren nach Anspruch 1, wobei die entzweigte Stärke in Schritt (c) durch Säure- oder Enzymhydrolyse weiterbehandelt wird, bevor man das resistente Stärkeprodukt isoliert.

5. Verfahren zur Herstellung eines mindestens etwa 15 % resistente Stärke enthaltenden resistenten Stärkeprodukts, das im wesentlichen aus den Schritten
(a) Herstellen eines wäßrigen, einen Feststoffgehalt von 15 bis 40 % aufweisenden Breis einer Stärke, die mindestens 40 % Amylose enthält;
(b) Gelieren des Stärkebreis bei einer Temperatur von 120-175 °C;
(c) Zugeben einer wirksamen Menge eines entzweigenen Enzyms zur gelierten Stärke, um die 1,6-glucosidischen Bindungen der Stärkemoleküle zu hydrolysieren;
(d) Zugeben einer wirksamen Menge eines anorganischen Salzes zur entzweigten Stärke, um das resistente Stärkeprodukt auszufallen;
(e) inkubieren des Gemisches aus Stärke und Salz bei Temperaturen zwischen 50°-100 °C;
(f) Auffangen des ausgefällten resistenten Stärkeprodukts
besteht.

6. Verfahren nach Anspruch 5, wobei die Stärke in Schritt (a) eine entfettete Stärke oder eine chemisch modifizierte Stärke ist.

7. Verfahren nach Anspruch 5, wobei das entzweigende Enzym aus der aus Pullulanase und Isoamylase bestehenden Gruppe ausgewählt wird.

8. Verfahren nach Anspruch 5, wobei das anorganische Salz der entzweigten Stärke in einer Menge von 10%-50 %, bezogen auf die Feststoffe in der Stärke, zugegeben wird.

9. Verfahren nach Anspruch 5, wobei die anorganischen Salze aus der aus Ammoniumsulfat, Natriumsulfat, Magnesiumsulfat und Natriumchlorid bestehenden Gruppe ausgewählt werden.

10. Verfahren nach Anspruch 5, wobei das kristallisierte Stärkeprodukt von Schritt (a) durch Säure- oder Enzymhydrolyse weiterbehandelt wird, bevor man das resistente Stärkeprodukt isoliert.

11. Resistentes Stärkeprodukt, gekennzeichnet durch eine Schmelzendotherme mit einem Peak im Bereich von 115°-135 °C.

12. Resistentes Stärkeprodukt nach Anspruch 11, das ferner durch eine Wasserlöslichkeit bei Raumtemperatur von mehr als 7 % gekennzeichnet ist.

## Revendications

1. Procédé pour la préparation d'un produit à base d'amidon résistant, contenant au moins environ 15 % d'amidon résistant, qui consiste essentiellement en les étapes suivantes :
(a) préparation d'une suspension aqueuse d'un amidon qui contient au moins 40 % d'amylose, à une teneur en matières solides de 15 % à 40 % ;
(b) gélatinisation de la suspension d'amidon à une température de 120° à 175°C ;
(c) addition d'une quantité efficace d'un enzyme déramifiant à l'amidon gélatinisé pour hydrolyser les liaisons 1,6-glucosidiques des molécules d'amidon ;
(d) isolement de l'amidon résistant déramifié, par séchage ou extrusion.

2. Procédé suivant la revendication 1, dans lequel l'amidon dans l'étape (a) est un amidon dégraissé ou un amidon modifié chimiquement.

3. Procédé suivant la revendication 1, dans lequel l'enzyme déramifiant est choisi dans le groupe consistant en pullulanase et iso-amylase.

4. Procédé suivant la revendication 1, dans lequel l'amidon déramifié dans l'étape (c) est traité en outre par hydrolyse acide ou hydrolyse enzymatique avant d'isoler le produit à base d'amidon résistant.

5. Procédé pour la préparation d'un produit à base d'amidon résistant, contenant au moins environ 15 % d'amidon résistant, qui consiste essentiellement en les étapes suivantes :
(a) préparation d'une suspension aqueuse d'un amidon contenant au moins 40 % d'amylose, à une teneur en matières solides de 15 % à 40 % ;
(b) gélatinisation de la suspension d'amidon, à une température de 120° à 175°C ;
(c) addition d'une quantité efficace d'un enzyme déramifiant à l'amidon gélatinisé pour hydrolyser les liaisons 1,6-glucosidiques des molécules d'amidon ;
(d) addition d'une quantité efficace d'un sel inorganique à l'amidon déramifié pour précipiter le produit à base d'amidon résistant ;
(e) mise en incubation du mélange d'amidon et de sel à des températures comprises dans l'intervalle de 50° à 100°C ;
(f) recueillir le produit à base d'amidon résistant, précipité.

6. Procédé suivant la revendication 5, dans lequel l'amidon dans l'étape (a) est un amidon dégraissé ou un amidon modifié chimiquement.

7. Procédé suivant la revendication 5, dans lequel l'enzyme déramifiant est choisi dans le groupe consistant en pullulanase et iso-amylase.

8. Procédé suivant la revendication 5, dans lequel le sel inorganique est ajouté à l'amidon déramifié en une quantité de 10 % à 50 % en poids des matières solides de l'amidon.

9. Procédé suivant la revendication 5, dans lequel les sels inorganiques sont choisis dans le groupe consistant en sulfate d'ammonium, sulfate de sodium, sulfate de magnésium et chlorure de sodium.

10. Procédé suivant la revendication 5, dans lequel le produit consistant en amidon cristallisé de l'étape (a) est traité en outre par hydrolyse acide ou hydrolyse enzymatique avant d'isoler le produit à base d'amidon résistant.

11. Produit à base d'amidon résistant, caractérisé par un comportement endothermique de fusion présentant un pic dans la plage de 115° à 135°C.

12. Produit à base d'amidcn résistant suivant la revendication 11, caractérisé en outre par une solubilité dans l'eau à température ambiante supérieure à 7 %.
